# DEMANDE DE BREVET EUROPEEN

(11) **EP 1 475 072 A1**
(43) Date de publication de la demande: **10.11.2004**
(21) Numéro de dépôt: 03104916.6
(22) Date de dépôt: 22.12.2003
(51) Int. Cl.: A61K 7/04

(54) **Utilisation de dialdylsulfones dans des compositions cosmetiques de soin des ongles pour favoriser la pousse de l'ongle**

(30) Priorité: 24.12.2002 FR 0216651
(71) Demandeur: L'OREAL, 75008 Paris (FR)
(72) Inventeur: Picard-Lesboueyries, Elisabeth, 78140, VELIZY (FR); Bernard, Pascale, 94370, SUCY-EN-BRIE (FR)
(74) Mandataire: Poulin, Gérard

(57) **Abrégé**

Utilisation d'au moins une dialkylsulfone de formule (I) :

R₁-SO₂-R₂ (I)

dans laquelle R₁ et R₂ identiques ou différents représentent un groupe alkyle linéaire ou ramifié de 1 à 4 atomes de carbone, dans une composition cosmétique de soin des ongles, appliquée sur les ongles, pour favoriser la pousse des ongles.

## Description

La présente invention concerne l'utilisation de dialkylsulfones et, en particulier, du méthylsulfonylméthane dans des compositions de soin des ongles, telles que des vernis à ongle pour favoriser, activer, la pousse de l'ongle, accroître la vitesse de pousse de l'ongle.

Les produits de soin des ongles, tels que les vernis à ongle peuvent contenir divers produits biologiquement actifs qui ont pour but notamment de renforcer ou de durcir les ongles.

Ainsi, sont connus et commercialisés des produits de soin des ongles qui contiennent de la silice ou du formaldéhyde, en tant qu'agent durcisseur des ongles.

D'autres produits de soin des ongles contiennent des complexes multivitaminés, du fluor, de la vitamine E, ou bien du calcium.

Il n'existe aucune composition de soin des ongles qui ait un effet réel sur la pousse de l'ongle et qui, éventuellement, renforce également les ongles.

Il a été mis en évidence, selon la présente invention, que l'incorporation de dialkylsulfones dans les compositions cosmétiques de soin pour les ongles permettait de favoriser la pousse des ongles ; en d'autres termes, d'accélérer la pousse de l'ongle, d'accroître la vitesse de pousse par rapport à une vitesse de pousse normale.
En particulier, il a été mis en évidence que l'incorporation de dialkylsulfones dans les compositions cosmétiques de soin pour les ongles permettait de favoriser la pousse des ongles mous.
Par ongle mou, on entend un ongle flexible, qui se courbe facilement lorsqu'on essaye de le plier.
Par ongle dur, on entend un ongle présentant une résistance lorsqu'on essaye de le plier.

L'invention a donc pour objet l'utilisation d'au moins une dialkylsulfone de formule (I) :

R₁-SO₂-R₂ (I)

dans laquelle R₁ et R₂ identiques ou différents représentent un groupe alkyle linéaire ou ramifié de 1 à 4 atomes de carbone, dans une composition cosmétique de soin des ongles, appliquée sur les ongles, pour favoriser la pousse des ongles.

Le groupe alkyle de R₁ et R₂ est choisi indépendamment parmi les groupes alkyle linéaire et ramifié de 1 à 4 atomes de carbone, tels que les groupes méthyle, éthyle, isopropyle, n-propyle, n-butyle, isobutyle et tertiobutyle.

De préférence, R₁ et R₂ sont identiques.

De préférence encore, R₁ et R₂ représentent tous deux un groupe méthyle et le composé de formule (I) est alors la diméthylsulfone ou méthylsulfonylméthane (MSM).

Les composés de formule (I) et, en particulier le MSM, sont des composés connus. Le MSM [67-71-0] a l'avantage d'être un produit sans odeur, blanc, cristallin, qui a un point de fusion de 109°C et un point d'ébullition de 238°C.

Le MSM est présent dans la plupart des végétaux verts, du microplancton et des algues.

C'est un composé naturel que l'on trouve dans les tissus nerveux, la peau, les cheveux et les articulations.

Le MSM a déjà été utilisé dans des compositions cosmétiques et des produits de régime.

Ainsi, le document US-A-4 296 130 décrit-il une composition cosmétique pour la peau, les ongles comprenant du MSM dans des solutions aqueuses ou non aqueuses ou en émulsion. Le MSM utilisé dans des produits de manucure permet d'améliorer la résistance des ongles et d'obtenir des ongles moins cassants.

L'exemple 13 de ce document est une formulation aqueuse contenant 10 % de MSM et 50 % d'eau appliquée sur l'ongle avec un coton. On laisse agir 15 minutes. Les ongles sont résistants, moins cassants.

L'exemple 14 de ce document est un vernis solvant (acétate d'éthyle ou acétate d'amyle) contenant 5 % en poids de MSM. L'application de ce vernis rend les ongles moins cassants.

Dans l'exemple 15 de ce document, des produits pour éliminer les vernis à ongles sont préparés en combinant un solvant à vernis classique avec 5 à 10 % de MSM. On observe que les ongles deviennent moins cassants.

Il est donc connu de ce document de formuler le MSM, sans qu'il soit nécessairement associé avec le carbamide et/ou le DMSO, dans un vernis solvant ou en milieu aqueux pour améliorer la résistance des ongles et les rendre moins cassants, mais ce document ne mentionne ni ne suggère en aucune manière que le MSM ait une quelconque influence sur la pousse des ongles.

Le document EP-A-0 200 526 concerne l'utilisation du MSM dans des aliments, en tant que source de soufre, et dans la préparation d'agents thérapeutiques, notamment antiarthritiques, analgésiques, antiparasites et anticrampes administrés par voie orale à des animaux ou des êtres humains.

Ce document décrit à l'exemple 6 que l'ingestion par voie orale de MSM, sous forme d'une solution aqueuse à 5 %, favorise, stimule, la pousse de l'ongle selon un test effectué sur des chiens.

Si, ce document enseigne donc que l'administration du MSM par voie orale favorise la pousse de l'ongle, il ne mentionne, ni ne suggère que l'application topique du MSM sur l'ongle peut conduire à un tel résultat. On sait qu'on ne peut en rien déduire, en se basant sur l'efficacité d'un produit administré par voie orale, que ce produit aura la même action, le même effet, administré par une voie différente, par exemple par voie topique.

Ainsi, l'effet montré d'un produit administré par voie orale ne préjuge en rien de l'effet de ce même produit par voie topique, par exemple sur l'ongle.

Le document US-A-5 785 959 décrit une composition de renforcement des ongles comprenant :
- 0,001-20 % d'un agent thiolé réticulant ;
- 0,1-60 % d'un agent de « permeation/binding » (en particulier des hydroxy acides carboxyliques) ;
- 0,001-20 % d'agent chélatant.

Parmi les agents thiolés réticulants, est citée la diméthylsulfone ou méthylsulfonylméthane (MSM).

La composition peut contenir de l'eau ou des huiles. Elle peut aussi être anhydre, notamment sous forme de vernis à ongles.

Les exemples enseignent que la composition renforçatrice des ongles peut être un vernis solvant (exemple 3) avec de la nitrocellulose et de l'acétate d'éthyle ou de butyle (exemple 2) ou un vernis à l'eau avec un latex acrylique.

En conclusion, ce document suggère donc l'emploi du MSM formulé dans un vernis solvant ou un vernis à l'eau ou avec un latex, pour renforcer les ongles, les propriétés renforçatrices n'étant pas autrement détaillées.

Ce document ne mentionne, ni ne suggère que le MSM ait une influence quelconque sur la pousse des ongles.

L'utilisation des composés de formule (I) dans une composition de soin des ongles, appliquée sur l'ongle, par voie topique, pour favoriser, activer, la pousse de l'ongle n'est donc ni mentionnée, ni suggérée dans l'art antérieur.

En outre, les composés de formule (I) utilisés dans des compositions de soin pour les ongles permettent aussi de rendre les ongles plus durs, se dédoublant moins, c'est-à-dire que l'utilisation des composés de formule (I) dans des compositions de soin pour les ongles permet aussi de renforcer les ongles, d'améliorer l'aspect global de l'ongle.

De plus, les propriétés cosmétiques des compositions dans lesquelles le composé de formule (I) est utilisé ne sont pas affectées et demeurent excellentes. En particulier, quelle que soit la forme de la composition, les propriétés, telles que la brillance, le tendu de film et la tenue sont conservées en présence d'un composé de formule (I), tel que le MSM.

La composition cosmétique de soin des ongles comprend le ou les composés de formule (I) dans un milieu cosmétiquement acceptable.

La teneur en composé (s) de formule (I) est généralement de 0,05 à 10 % en poids, de préférence de 0,1 à 4% en poids du poids total de la composition.

Par milieu cosmétiquement acceptable, on entend un milieu compatible avec les ongles.

Les compositions selon l'invention peuvent se présenter sous une forme quelconque à la condition qu'elle soit appliquée sur l'ongle : il pourra s'agir d'une simple solution aqueuse ou dans un solvant, d'une crème de soin des ongles, d'un dissolvant pour vernis à ongles, d'un vernis à ongle.

De préférence, la composition cosmétique de soin des ongles est un vernis à ongles.

Le milieu cosmétiquement acceptable de la composition peut comprendre un milieu aqueux ou un milieu solvant organique.

Lorsque la composition comprend un milieu solvant organique comprenant un solvant organique, on peut citer comme solvant organique utilisable dans la composition où est utilisée le composé de formule (I) :
- Les cétones liquides à température ambiante, telles que la méthyléthylcétone, la méthylisobutylcétone, la diisobutylcétone, l'isophorone, la cyclohexanone, l'acétone ;
- les alcools liquides à température ambiante, tels que l'éthanol, l'isopropanol, le diacétone alcool, le 2-butoxyéthanol, le cyclohexanol ;
- les glycols liquides à température ambiante, tels que l'éthylène glycol, le propylène glycol, le pentylène glycol, le glycérol ;
- les éthers de propylène glycol liquides à température ambiante, tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total), tels que l'acétate d'éthyle, l'acétate de méthyle, l'acétate de propyle, l'acétate de n-butyle, l'acétate d'isopentyle ;
- les alcanes liquides à température ambiante, tels que le décane, l'heptane, le dodécane, le cyclohexane ;
- les aldéhydes liquides à température ambiante, tels que le benzaldéhyde, l'acétaldéhyde ; et
- leurs mélanges.

De préférence, le solvant est choisi parmi les esters à chaîne courte (ayant de 3 à 8 atomes de carbone au total), les alcools liquides à température ambiante, et leurs mélanges.

La composition à milieu solvant organique peut comprendre, en outre, de l'eau, notamment à une teneur allant de 0,1 à 10 % en poids, par rapport au poids total de la composition.

Lorsque la composition, telle qu'un vernis à ongles comprend un milieu solvant organique, le solvant organique peut être présent en une teneur allant de 25 à 94,9 % en poids, par rapport au poids total de la composition, et de préférence de 60 % à 90 % en poids.

Lorsque la composition, telle qu'un vernis à ongles, contient un milieu aqueux, ce dernier peut être constitué essentiellement d'eau (notamment le milieu aqueux est de l'eau) ou bien encore peut comprendre un mélange d'eau et d'un ou plusieurs solvants miscible(s) à l'eau, comme les monoalcools inférieurs ayant de 1 à 5 atomes de carbone, tels que l'éthanol, les glycols ayant de 2 à 8 atomes de carbone, les cétones en C₃-C₄ et les aldéhydes en C₂-C₄.

La teneur en eau de la composition peut aller de 25 à 95 % en poids par rapport au poids total de la composition, de préférence de 50 à 90 % en poids et mieux de 60 à 85 % en poids.

Notamment, lorsque la composition est une composition de vernis à ongles, elle peut contenir au moins un polymère filmogène en une proportion allant généralement de 1 à 60 % en poids par rapport au poids total de la composition et mieux de 5 à 45 % en poids dans un milieu solvant organique ou un milieu aqueux, tel que défini ci-dessus.

Dans la présente demande, on entend par « polymère filmogène » un polymère apte à former à lui seul ou en présence d'un agent plastifiant éventuel, un film isolable. Le polymère filmogène peut être solubilisé ou dispersé sous forme de particules dans le milieu cosmétiquement acceptable de la composition.

Le polymère filmogène peut être choisi parmi les polymères radicalaires, les polycondensats et les polymères d'origine naturelle.

Le polymère filmogène peut être choisi notamment dans le groupe formé par les polymères vinyliques et les acryliques, les polyuréthanes, les polyesters, les résines alkydes, les résines époxyesters, les polymères cellulosiques, tels que la nitrocellulose, les esters de cellulose comme l'acétate de cellulose, l'acétopropionate de cellulose, l'acétobutyrate de cellulose, les résines résultant de la condensation de formaldéhyde avec une arylsulfonamide et leurs mélanges.

Lorsque la composition, en particulier sous la forme d'un vernis à ongles, comprend un milieu solvant organique, le polymère filmogène peut être choisi notamment parmi les résines alkydes, acryliques et/ou vinyliques, les polyuréthanes et les polyesters, les celluloses et dérivés cellulosiques, telles que la nitrocellulose, les esters de cellulose, tels que l'acétate de cellulose, l'acétopropionate de cellulose, l'acétobutyrate de cellulose, les résines résultant de la condensation de formaldéhyde avec une arylsulfonamide et leurs mélanges.

Lorsque la composition, telle qu'un vernis à ongle, comprend un milieu aqueux, alors le polymère filmogène est généralement présent sous la forme de particules en dispersion dans le milieu aqueux formant ainsi un latex ou pseudo latex.

Parmi les polymères filmogènes susceptibles d'être utilisés en milieu aqueux, on peut citer les polyuréthanes, par exemple anioniques, les polyesters-polyuréthanes, les polyéther-polyuréthanes, les polymères radicalaires, notamment de type acrylique, acrylique styrène et/ou vinylique, les polyesters, les résines alkydes, seuls ou en mélange.

La dispersion peut également comprendre un polymère associatif de type polyuréthane ou une gomme naturelle, telle que la gomme xanthane.

Comme polymère en dispersion aqueuse, on peut citer les dispersions de polymères acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la Société ZENECA, DOW LATEX 432®, par la Société DOW CHEMICAL. On peut également employer des dispersions aqueuses de polyuréthane, et notamment les polyester-polyuréthanes vendus sous les dénominations « AVALURE UR-405® », « AVALURE UR-410® », « AVALURE UR-425® », « SANCURE 2060® » par la Société GOODRICH et les polyéther-polyuréthanes vendus sous les dénominations « SANCURE 878® » par la Société GOODRICH, « NEOREZ R-970® » par la Société AVECIA.

Le polymère filmogène peut être présent dans la composition selon l'invention en une teneur en matières sèches, efficace pour l'obtention d'un film allant généralement de 5 % à 60 % en poids, par rapport au poids total de la composition, et mieux de 10 % à 40 % en poids.

Pour améliorer les propriétés filmogènes de la composition, notamment du vernis à ongle, dans laquelle est utilisée le composé de formule (I), un agent auxiliaire de filmification peut être prévu.

Un tel agent auxiliaire de filmification peut être choisi parmi tous les composés connus de l'homme du métier, comme étant susceptibles de remplir la fonction recherchée, et être notamment choisi parmi les agents plastifiants.

En outre, lorsque la composition de base selon l'invention comprend un polymère filmogène sous forme de particules dispersées dans un milieu aqueux, l'agent auxiliaire de filmification peut aussi être choisi parmi les agents de coalescence.

La composition peut comprendre, en outre, au moins un agent plastifiant. En particulier, on peut citer, seuls ou en mélange, les plastifiants usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, des tartrates, des phosphates, des sébaçates ;
- des dérivés oxyéthylénés, tels que les huiles oxyéthylénées, notamment les huiles végétales, telles que l'huile de ricin ;
- leurs mélanges.

La quantité de plastifiant peut être choisie par l'homme du métier sur base de ses connaissances générales, de manière à obtenir une composition ayant des propriétés cosmétiquement acceptables. La teneur en plastifiant peut, par exemple, aller de 0,1 % à 15 % en poids, par rapport au poids total de la composition, et de préférence de 0,5 % à 10 % en poids.

La composition peut comprendre une matière colorante qui peut être choisie parmi les composés pulvérulents et/ou les colorants solubles dans le milieu de la composition. La matière colorante peut être présente en une teneur allant de 0,01 % à 10 % en poids, par rapport au poids total de la composition. Les composés pulvérulents peuvent être choisis parmi les pigments et/ou les nacres et/ou les paillettes, habituellement utilisés dans les compositions cosmétiques, telles que les vernis à ongles.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique, les pigments métalliques comme l'aluminium ou le bronze. Parmi les pigments organiques, on peut citer le noir de carbone, les pigments de type D & C, et les laques à base de carmin de cochenille, de baryum, strontium, calcium, aluminium, la guanine.

Les pigments nacrés peuvent être choisis parmi les pigments nacrés blancs, tels que le mica recouvert de titane, ou d'oxychlorure de bismuth, les pigments nacrés colorés, tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les paillettes peuvent être choisies parmi celles en résine acrylique, polyester, polyéthylène téréphtalate, en aluminium.

Les colorants sont, par exemple, le rouge Soudan, le DC Red 17, le DC Green 6, le β-carotène, l'huile de soja, le brun Soudan, le DC Yellow 11, le DC Violet 2, le DC Orange 5, le jaune quinoléine.

La composition peut comprendre, en outre, tout autre ingrédient, additif, utilisé couramment dans les compositions cosmétiques et connu de l'homme du métier comme étant susceptible d'être incorporé dans un telle composition, telle qu'un vernis à ongles.

De tels ingrédients, additifs, peuvent être choisis parmi les agents de coalescence, les agents épaississants, les conservateurs, les parfums, les huiles, les cires, les tensioactifs, les antioxydants, les agents antiradicaux libres, les agents d'étalement, les agents mouillants, les agents dispersants, les anti-mousse, les neutralisants, les stabilisants, les actifs choisis parmi les huiles essentielles, les filtres UV/solaires, les agents hydratants, les vitamines, les protéines, les céramides ; et leurs mélanges.

Bien entendu, l'homme du métier veillera à choisir ce ou ces éventuels composés complémentaires et/ou leur quantité, de manière telle que les propriétés avantageuses liées à l'utilisation dans la composition selon l'invention d'un composé de formule (I) ne soient pas, ou substantiellement pas altérées par l'adjonction envisagée.

La composition dans laquelle est utilisé le composé de formule (I), tel que le MSM, se présente sous une des formes déjà décrites plus haut. La composition peut être appliquée sur l'ongle par tout moyen adéquat, tel que pinceau, brosse, tampon, lingette, etc.

L'invention va maintenant être décrite en référence aux exemples suivants, donnés à titre illustratif et non limitatif.

### Description détaillée de modes de réalisation particuliers

Les exemples suivants 1 et 2 illustrent la préparation de compositions cosmétiques de soin des ongles dans lesquelles est incorporé un composé de formule (I), à savoir du MSM.

### Exemple 1

Vernis à l'eau contenant du MSM.

On prépare, une formule de vernis à l'eau contenant 89,47 % d'un latex de polyuréthane (CYDROTHANE HP 6000 de la société CYTEC), auquel on ajoute un agent de coalescence (dipropylène glycol monobutyl éther vendu par la société DOW sous le nom de « DOWANOL DPnB ») (1,1 %), puis des conservateurs (0,54 % de sodium methyl paraben et 0,4% de diazolidinyl urée), 0,05 % de tensioactif pour faciliter le pelage et l'étalement du film (polyfluorohexyl betaine tel que l'Amphoram® de la société CECA®), de l'éthanol (4,36 %), 0,09% de polyuréthane associatif (Serad® FX 1100 de la société SERVO DELDEN) et 2 % de MSM broyé au préalable mécaniquement au mortier. Pour accélérer la dissolution du MSM dans dans le mélange précédent, on peut chauffer l'ensemble au bain-marie (à environ 50°C).

### Exemple 2

Vernis solvant contenant du MSM.

On ajoute à de la nitrocellulose (10 g), préalablement mélangée à de l'acétate d'éthyle, 0,5 % de MSM ; puis après complète dissolution, on ajoute une résine du type alkyde, un plastifiant (acétyltributyle citrate commercialisé sous la référence Citroflex® par la société Pfizer) (taux de résine et de plastifiant égal à 15 g) et enfin on complète avec les deux solvants (acétate d'éthyle et de butyle qsq 100 g).

Bien entendu, les deux formulations des exemples 1 et 2 peuvent contenir des pigments, colorants, nacres et autres additifs.

## Revendications

1. Utilisation d'au moins une dialkylsulfone de formule (I) :
R₁-SO₂-R₂ (I)
dans laquelle R₁ et R₂ identiques ou différents représentent un groupe alkyle linéaire ou ramifié de 1 à 4 atomes de carbone, dans une composition cosmétique de soin des ongles, appliquée sur les ongles, pour favoriser la pousse des ongles.

2. Utilisation selon la revendication 1, dans laquelle le groupe alkyle de R₁ et R₂ est choisi indépendamment parmi les groupes méthyle, éthyle, isopropyle, n-propyle, n-butyle, isobutyle et tertiobutyle.

3. Utilisation selon l'une quelconque des revendications précédentes, dans laquelle R₁ et R₂ sont identiques.

4. Utilisation selon la revendication 3, dans laquelle R₁ et R₂ représentent tous deux un groupe méthyle et le composé de formule (I) est la diméthylsulfone ou méthylsulfonylméthane (MSM).

5. Utilisation selon l'une quelconque des revendications précédentes, pour, en outre, rendre les ongles plus durs.

6. Utilisation selon l'une quelconque des revendications précédentes, dans laquelle la teneur en composé de formule (I) est de 0,05 à 10 % en poids, de préférence de 0,1 à 4 % en poids, du poids total de la composition.

7. Utilisation selon l'une quelconque des revendications précédentes, dans laquelle la composition comprend le ou les composé(s) de formule (I) dans un milieu cosmétiquement acceptable.

8. Utilisation selon l'une quelconque des revendications précédentes, dans laquelle la composition comprend un milieu solvant organique comprenant un solvant organique.

9. Utilisation selon la revendication 8, dans laquelle le solvant organique est choisi parmi :
- les cétones liquides à température ambiante, telles que la méthyléthylcétone, la méthylisobutylcétone, la diisobutylcétone, l'isophorone, la cyclohexanone, l'acétone ;
- les alcools liquides à température ambiante, tels que l'éthanol, l'isopropanol, le diacétone alcool, le 2-butoxyéthanol, le cyclohexanol ;
- les glycols liquides à température ambiante, tels que l'éthylène glycol, le propylène glycol, le pentylène glycol, le glycérol ;
- les éthers de propylène glycol liquides à température ambiante, tels que le monométhyléther de propylène glycol, l'acétate de monométhyl éther de propylène glycol, le mono n-butyl éther de dipropylène glycol ;
- les esters à chaîne courte ayant de 3 à 8 atomes de carbone au total, tels que l'acétate d'éthyle, l'acétate de méthyle, l'acétate de propyle, l'acétate de n-butyle, l'acétate d'isopentyle ;
- les alcanes liquides à température ambiante, tels que le décane, l'heptane, le dodécane, le cyclohexane ;
- les aldéhydes liquides à température ambiante, tels que le benzaldéhyde, l'acétaldéhyde ; et
- leurs mélanges.

10. Utilisation selon l'une quelconque des revendications 8 à 9, dans laquelle le solvant organique est choisi parmi les esters à chaîne courte, les alcools liquides à température ambiante, et leurs mélanges.

11. Utilisation selon l'une quelconque des revendications 8 à 10, dans laquelle le solvant organique est présent à une teneur allant de 25 à 94,9 % en poids, par rapport au poids total de la composition.

12. Utilisation selon l'une quelconque des revendications 1 à 7, dans laquelle la composition comprend un milieu aqueux.

13. Utilisation selon la revendication 12, dans laquelle le milieu aqueux est constitué essentiellement d'eau ou d'un mélange d'eau et d'un ou plusieurs solvants miscibles à l'eau.

14. Utilisation selon l'une quelconque des revendications 12 à 13, dans laquelle la teneur en eau de la composition est de 25 à 95 % en poids, de préférence de 50 à 90 % en poids et mieux de 60 à 85 % en poids.

15. Utilisation selon l'une quelconque des revendications précédentes, dans laquelle la composition comprend au moins un polymère filmogène en une proportion de 1 à 60 % en poids par rapport au poids total de la composition.

16. Utilisation selon la revendication 15, dans laquelle le polymère filmogène est choisi parmi les polymères radicalaires, les polycondensats et les polymères d'origine naturelle.

17. Utilisation selon l'une quelconque des revendications 15 et 16, dans laquelle la composition comprend un milieu solvant organique et le polymère filmogène est choisi parmi les résines alkydes, acryliques et/ou vinyliques, les polyuréthanes et les polyesters, les celluloses et dérivés cellulosiques, telles que la nitrocellulose, les esters de cellulose, tels que l'acétate de cellulose, l'acétopropionate de cellulose, l'acétobutyrate de cellulose, les résines résultant de la condensation de formaldéhyde avec une arylsulfonamide, et leurs mélanges.

18. Utilisation selon l'une quelconque des revendications 15 et 16, dans laquelle la composition comprend un milieu aqueux et le polymère filmogène est présent sous la forme de particules en dispersion dans le milieu aqueux.

19. Utilisation selon la revendication 18, dans laquelle le polymère filmogène est choisi parmi les polyuréthanes, par exemple anioniques, les polyesters-polyuréthanes, les polyéther-polyuréthanes, les polymères radicalaires, notamment de type acrylique, acrylique styrène et/ou vinylique, les polyesters, les résines alkydes, seuls ou en mélange.

20. Utilisation selon l'une quelconque des revendications 15 à 19, dans laquelle la composition comprend un agent auxiliaire de filmification.

21. Utilisation selon l'une quelconque des revendications précédentes, dans laquelle la composition comprend au moins un agent plastifiant.

22. Utilisation selon l'une quelconque des revendications précédentes, dans laquelle la composition comprend une matière colorante.

23. Utilisation selon la revendication 22, dans laquelle la matière colorante est choisie parmi les composés pulvérulents et/ou les colorants solubles dans le milieu de la composition.

24. Utilisation selon l'une quelconque des revendications précédentes, dans laquelle la composition comprend, en outre, un additif choisi parmi les agents de coalescence, les agents épaississants, les conservateurs, les parfums, les huiles, les cires, les tensioactifs, les antioxydants, les agents antiradicaux libres, les agents d'étalement, les agents mouillants, les agents dispersants, les anti-mousse, les neutralisants, les stabilisants ; les actifs choisis parmi les huiles essentielles, les filtres UV/solaires, les agents hydratants, les vitamines, les protéines, les céramides ; et leurs mélanges.

25. Utilisation selon l'une quelconque des revendications précédentes, dans laquelle la composition se présente sous la forme d'une solution aqueuse ou dans un solvant, d'une crème de soin pour les ongles, d'un vernis à ongle, ou d'un dissolvant pour vernis à ongle.

26. Utilisation selon la revendication 24, dans laquelle la composition est un vernis à ongles.
